# EUROPEAN PATENT SPECIFICATION

(11) **EP 2 162 180 B1**
(45) Date of publication and mention of the grant of the patent: **21.09.2011**
(21) Application number: 07852147.3
(22) Date of filing: 21.12.2007
(51) Int. Cl.: A61N 1/05

(54) **MEDICAL IMPLANTABLE LEAD AND METHOD FOR MANUFACTURING THE SAME**
MEDIZINISCHE IMPLANTIERBARE LEITUNG UND VERFAHREN ZU IHRER HERSTELLUNG
FIL MEDICAL IMPLANTABLE ET SON PROCEDE DE FABRICATION

(30) Priority: 28.06.2007 WO PCT/SE2007/000634
(43) Date of publication of application: 17.03.2010
(60) Divisional application: 11152316.3
(73) Proprietor: St. Jude Medical AB, 175 84 Järfälla (SE)
(72) Inventor: HILL, Rolf, S-175 55 Järfälla (SE); STEGFELDT, Olof, S-138 37 Älta (SE)
(86) International application number: PCT/SE2007/001153
(87) International publication number: WO 2009/002240

(56) References cited:
- WO-A1-01/45791
- WO-A1-02/087689
- WO-A1-2006/014757
- US-A- 4 540 236
- US-A- 4 628 943
- US-A- 5 716 390
- US-A- 5 741 321
- US-B1- 6 463 334
- US-B1- 6 489 562

## Description

The invention relates to a medical implantable lead for monitoring and/or controlling of an organ inside a human or animal body, comprising two electrical conductors, each connected to a respective electrode for receiving or transmitting of electrical signals from or to the organ, and a tubular header in a distal end of the lead, wherein one electrode is positioned in the outermost distal end of the lead whereas the other is a ring formed electrode, which is mounted on the outside of the header by means of a coupling.

The invention also relates to a method for manufacturing of such a medical implantable lead.

### Background of the invention

Medical implantable leads of the above kind are well known in the art. One common field of application is for monitoring and/or controlling of the function of a human or animal heart, in which case it is inserted into the body and attached with a distal end to the heart by means of a fixating member, such as a helix which can be screwed into the tissue. A proximal end of the lead is connected to an electronic device, for performing the monitoring and/or controlling, such as a pacemaker or an implantable cardiac defibrillator.

The most distal of the electrodes can be formed as a passive electrode, in which case it is formed in the end surface of the lead and bears against the surface of the heart while being fixated to the heart wall by means of fins, tines or the like, which during implantation engages the trabecular network inside the heart and subsequently are overgrown by a layer of tissue. The distal electrode can also be of an active type, which is used both for monitoring/controlling and for fixation in that the electrode is inserted into and is engaged with the tissue, such as for example a tip provided with barbs or a helix which is screwed into the tissue. The most proximal of the electrodes is formed as a ring on the outside of the lead and positioned on a suitable distance from the distal end. The distance from the distal end is determined by the desired amplitude of the signals and an acceptable interference level.

Normally, the ring electrode is positioned at the proximal end of a so called header, which is formed as a tubular and rigid housing positioned in the distal end of the lead and adapted to carry the electrodes and the fixating member, e.g. accommodating the helix inside an inner bore of the header. The most common way to accomplish the mounting of the ring electrode to the header, is to arrange a coupling member between the header and the ring electrode which is partly inserted into the tubular bore from the proximal end of the header and partly into the ring electrode. In this way a distance is achieved between the ring electrode and the distal electrode, which is suitable for most applications and the outer surface of the ring electrode can be mounted flush with the outer surface of the header or a sleeve covering the header such that the maximum diameter of the lead can be made advantageously small. The fixation of the ring electrode in relation to the coupling member is normally achieved by an adhesive, which has to result that the assembled header, coupling member and ring electrode have to be held immovable and fixated during the time for curing of the adhesive. This is a disadvantage since it has to effect that the manufacturing process of the lead has to be suspended for a while. Moreover, this manufacturing method of the lead is also disadvantageous in that there is not provided any fixed position for the ring electrode in relation to the header. This has to effect that the position and orientation of the ring electrode in relation to the header can vary and the production yield loss of this kind of leads is comparatively high due to failures when interconnecting the ring electrode and the header.

Normally, the header as well as the coupling member are manufactured of an electrically conducting material, such as titanium, which means that a separate electrically insulating layer must be provided since the ring electrode must be electrically insulated in relation to the header. However, in prior art is also known headers made of an electrical insulating material, such as PEEK (polyetheretherketone). In this case there is no need for an electrical insulation between the ring electrode and the header. In the prior art it is also known to place the ring electrode on the outside around and bearing directly against a header of an electrically insulating material. In this way it is possible to arrange a defined position for the ring electrode on the header. However, an adhesive must be used to finally fixate the ring electrode on the header, which is a disadvantage in that the adhesive has to be applied carefully in a correct position, in order to ensure proper function of the completed lead, and requires some time for curing during which time the ring electrode may be dislodged from its position. Another disadvantage with such a design of the lead is that the ring electrode must be connected electrically, by means of a separate conductor, to a conductor in the lead. This adds additional components and manufacturing steps to the manufacturing process.

A lead with another design of the connection between a ring electrode and a member accomodating a helix to be screwed into tissue is disclosed in US 4,628,943. More specifically, US 4,628,943 relates to a bipolar screw-in pacing lead. The lead comprises a lead body, a proximal terminal electrode assembly located at the proximal end of the lead body and a distal electrode assembly located at the distal end of the lead body. The distal electrode assembly comprises a cathode ring electrode, a corkscrew anode electrode and an internal insulative tubular sleeve member. The cathode ring electrode has an integral rearwardly extending sleeve including a stepped proximal end portion. The insulative tubular sleeve member, has a distal sleeve portion that is situated within the ring electrode and accomodates the corkscrew anode electrode. Furthermore, the insualtive tubular sleeve member has a rearwardly extending hub portion including an annular rib and a barrel portion. The annular rib of the insulative tubular sleeve member is received in the proximal end portion of the sleeve of the ring electrode against the stepped portion with an annular space defined between the end portion of the sleeve of the ring electrode and the barrel portion of the insulative tubular sleeve member. The annular space is arranged for receiving, preferably with an interference fit, a distal end of a coiled wire conductor for connection with the sleeve of the ring electrode.

### Summary of the invention

An object of the present invention is to improve medical implantable leads according to prior art and provide a medical implantable lead which can be manufactured to a low cost. At least this object is achieved by a medical implantable lead according to claim 1.

The invention also relates to a method for manufacturing of a medical implantable lead, having essentially the same object as above. At least this object is achieved by a method according to claim 5.

The basis of the invention is the insight that the above object can be achieved by manufacturing the header of an electrically insulating material and to integrate the coupling member and the header into one unitary piece, wherein the coupling member is formed as a coupling portion in the header and formed in such a way that the ring electrode can be instantly fixated to the header by means of a quick fixating connection. Even though it is possible to also use an adhesive for increased bonding strength and fluid tight sealing of the lead, it is in this way possible to fixate the ring electrode instantly without having to wait for the adhesive to cure before proceeding with subsequent manufacturing steps.

Furthermore, according to the invention the quick fixating connection is formed as a clamp connection, wherein the ring electrode is clamped around a coupling portion of the header. The clamp connection is carried out by punching locking tabs from the ring electrode, which engage in a coupling portion in form of recesses in the outer surface of the header and lock the ring electrode in relation to the header. This kind of clamp connection is advantageous in so far as it prevents the ring electrode from being displaced in the longitudinal direction as well being rotated in relation to the header. However, the clamp connection can be carried out also in other ways, such as a clamp connection where the circumference of the ring electrode is crimped around and pressed towards the header. A quick fixating connection in form of a clamp connection is also advantageous in so far as it easily can be used to locate the ring electrode with different desirable distance from the distal tip of the medical implantable lead, as is illustrated in two different embodiments hereinafter.

In the described and illustrated clamp connection embodiments, the ring electrode is formed such that a proximal sleeve portion, in the mounted state, reaches over an electrical conductor, which suitably is in form of an outer wire coil and which provides electrical connection to the proximal end of the medical implantable lead, such that the ring electrode also can be electrically connected to the outer wire coil by means of a clamp connection. In this way no separate connector is required for connection of the ring electrode to the outer wire coil. Preferably, the clamp connection of the ring electrode to the header as well as to the outer wire coil, is performed in the same operation. Also preferably, the connection of the outer wire coil to the header is performed in the same operation and with the same clamp connection as when connecting the ring electrode to the outer wire coil. However, it is to be understood that it would also be possible to perform the clamp connection of the ring electrode to the header and to the outer wire coil in separate operations and the outer wire coil can be attached to the header by some other means, such as by welding, for increased strength.

In the described and illustrated embodiments of the invention, the fixating member for fixating the lead to an organ inside a body, is formed as a helix, which is accommodated inside a bore in the header and which can be screwed out from the distal end and into the tissue of an organ. It is to be understood, however, that the fixating member can be formed in many other ways, such as for example as a sharp tip provided with barbs to be penetrated into the tissue or as fins or tines to be engaging the trabecular network inside a heart and to be subsequently overgrown by tissue. The lead can also be adapted for monitoring and/or controlling of other organs than a heart inside a body.

### Brief description of the drawings

A detailed description of a prior art embodiment as well as embodiments according to the invention, will hereinafter be given by way of example with reference to the drawings, in which:
- Fig 1: is a longitudinal section through a distal end of a lead according to a prior art embodiment;
- Fig 2: is a longitudinal section through a distal end of a lead according to a second embodiment of the invention in an unclamped state;
- Fig 3: is a longitudinal section according to fig 5 during clamping;
- Fig 4: is a longitudinal section through a completed lead according to figs 6 and 7; and
- Fig 5: is a longitudinal section through a lead according to a third em- bodiment of the invention.

### Detailed description of a prior art embodiment and embodiments according to the invention

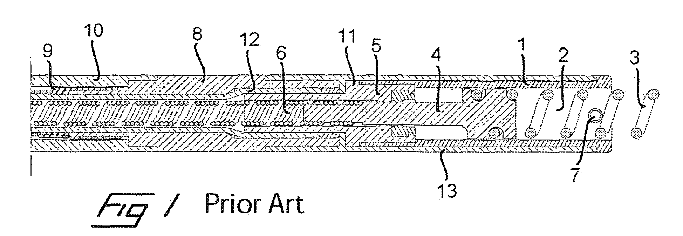

Reference is first made to fig 1 of the drawings, in which is illustrated a prior art embodiment of a distal end of a medical implantable lead. In the most distal end the lead comprises a tubular header 1 of an electrically conducting material such as titanium. In a distal bore 2 of the header, a helix 3 is accommodated, which is illustrated in a partially projecting state. The helix is mounted on a distal end of a shaft 4, which in its turn is journalled in a coupling member 5 such that it can be rotated as well as displaced in a longitudinal direction in relation to the coupling. The rotation of the helix 3 is actuated from the proximal end of the lead by rotation of an inner wire coil 6, which also functions as an electrical conductor from the proximal to the distal end. The helix 3 is in engagement with a post 7 on the inside of the header 1, such that when the inner wire coil 6 is rotated, the helix will be screwed out and advanced forward out from the header, which will have to effect that also the shaft 4 and the inner wire coil 6 will be advanced forward. When implanting the lead into a body, the helix is screwed out and into an organ such that the distal end is attached to the organ and the helix will function as an electrode in the organ. A second electrode is arranged in the proximal end of the header in form of a ring electrode 8, which is in electrical contact with the proximal end by means of an outer wire coil 9. Outside of the outer wire coil, an outer sleeve 10 is applied for protection of the lead.

Besides the function of rotationally bearing the shaft 4, the coupling member 5 also has the function of mounting and fixating the ring electrode 8. For this purpose, the coupling member 5 is formed with an intermediary section 11 having a comparatively large diameter, namely a diameter being equal to the outer diameter of the header 1, and end portions having a comparatively small diameter. When assembling the lead, one of the end portions of the coupling member is inserted into the header, as is illustrated in the drawing, and is interconnected by welding to the header, since also the coupling member is metallic. The other of the end portions is utilized for bearing of the ring electrode 8. However, since the header 1 and the coupling member 5 are metallic, they will have the same electrical potential as the shaft 4 and the helix 3. Accordingly, the ring electrode must be electrically insulated from the coupling member 5, which will have a different electrical potential. This is achieved by means of an inner sleeve 12, of silicone or the like, which surrounds the inner wire coil 6 and is extended up over a proximal end portion of the coupling member. The ring electrode 8 is positioned over the inner sleeve 12 and will accordingly be electrically insulated in relation to the coupling member 5, the header 1, the shaft 4 and the helix 3. However, the distal end of the ring electrode, is not allowed to come into electrical contact with the proximal end of the large diameter portion of the coupling member. Accordingly, when fixating the ring electrode to the coupling member by means of an adhesive, the ring electrode has to be held immovable, preferably in a fixture, in relation to the coupling member together with the header, for as long time as until the adhesive has been cured and the ring electrode is fixated. This means that the assembling of the lead has to be suspended for a while which is a disadvantage for the work flow when assembling the lead. Moreover, since there is no fixed position for the ring electrode on the coupling member, it frequently happens that the distance between the distal end of the ring electrode and the proximal end of the large diameter portion of the coupling becomes too small, in which case there is a risk for shortcircuiting, or too large in which case it is a risk that the assembled header, coupling and ring electrode does not fit with the rest of the lead components. It also happens that the header and the ring electrode becomes misaligned in relation to each other or that the insulating inner sleeve becomes damaged when passing the distal end of the ring electrode over the coupling member and the inner sleeve. All of these situations may lead to a rejection of the lead. Moreover, since the header is electrically conductive and has the same electrical potential as the distal electrode, i.e. the helix, it has to be covered by an electrically insulating layer in form of a header sleeve 13, of e.g. silicone, which normally is bonded directly onto the header such that the adhesive also penetrates into the gap between the proximal flange of the ring electrode and the large diameter portion 11 of the coupling member.

As is evident from the above description, the distal end of a medical implantable lead according to prior art, is composed of many different components and several different assembling steps are required for the manufacturing. Accordingly, it is desirable to provide a medical implantable lead which contains less component parts and requires fewer assembling steps for manufacturing, in order to reduce costs.

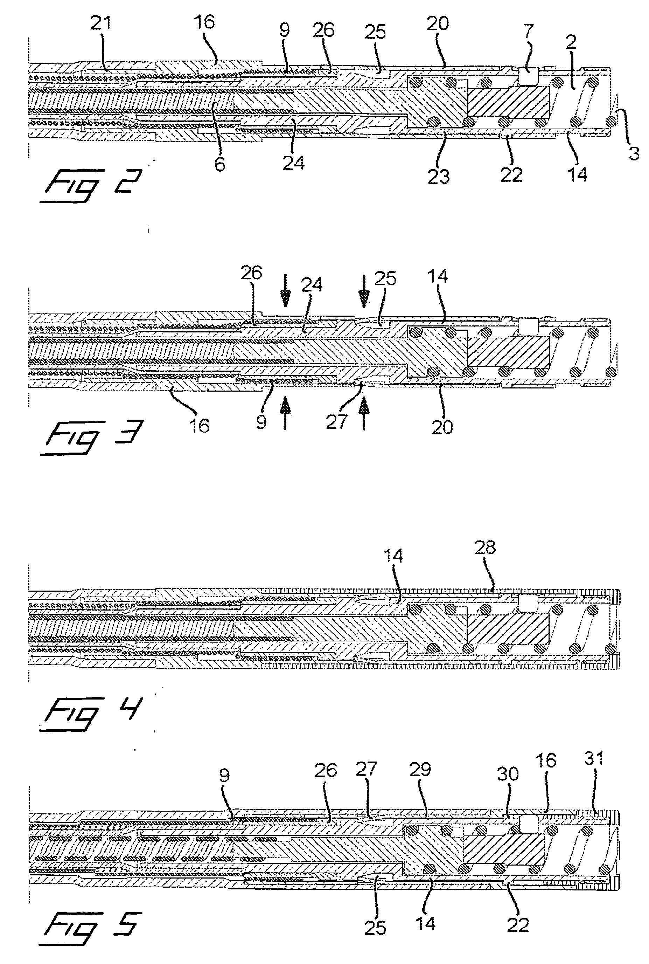

Reference is made to figs 2 to 4, in which are illustrated a second embodiment of the invention. In this embodiment the attachment between the header sleeve 14, being made of an electrically insulating material, and the ring electrode 16, is performed by means of a clamping connection. More precisely, the ring electrode 16 is provided with a distal sleeve portion 20 and a proximal sleeve portion 21 having slightly smaller outer diameters than the actual ring electrode 16. The header sleeve 14 in its turn, comprises an enlarged diameter portion 22 near its distal end, an intermediary support portion 23 for the distal sleeve portion 20 of the ring electrode, and a proximal support portion 24 with reduced diameter for the outer wire coil 9. The intermediary support portion 23 is formed with a coupling portion in form of recesses 25 around its periphery. When mounting the ring electrode 16 onto the header sleeve 14, the ring electrode 16 is slid onto the header sleeve 14 from its proximal end, until the end of the distal sleeve portion 20 abuts a proximal end surface of the enlarged diameter portion 22 of the header sleeve. Subsequently, the outer wire coil 9 together with a crimping sleeve 26 is inserted between the inner periphery surface of the ring electrode 16 and the proximal support portion 24 of the header sleeve until the distal end of the crimping sleeve 26 abuts a proximal end of the intermediary support portion 23. Thereafter, the assembly is positioned in a clamping tool by means of which clamping forces are applied over the recesses 25 and the proximal support portion 24, as is illustrated by the arrows in fig 3. This has to effect that locking tabs 27 will be punched out from the distal sleeve portion 20 of the ring electrode 16, which will be bent down into the recesses 25 and lock the ring electrode in relation to the header sleeve, and a proximal portion of the distal sleeve portion 20 will be pressed and crimped around the proximal support portion 24 such that the outer wire coil 9 and the clamping sleeve 26 will be pressed towards and secured in relation to the proximal support portion 24 by means of the clamp connection of the ring electrode 16. Finally, an outer protective sleeve 28 of an electrically insulating material, is arranged over the distal end of the header sleeve 14 and the distal sleeve portion 20 of the ring electrode, as is illustrated in fig 4.

In fig 5 is illustrated a third embodiment which, similar to the embodiment illustrated in figs 2 to 4, is formed with a quick fixating connection in form of a clamping connection. In this embodiment, however, the electrode surface of the ring electrode 16 is positioned close to the distal end of the header sleeve. This is accomplished by forming the ring electrode 16 with a proximal sleeve portion 29 which, at a transition section to the electrode surface, is formed with a stop surface 30 facing towards the distal end , which abuts against an enlarged diameter portion 22 of the header sleeve 14. The raised portion forming the electrode surface of the ring electrode 16, rests partly on the enlarged diameter portion 22 and partly on a silicone cap 31 in the end of the header sleeve. In all other respects, this embodiment is similar to the embodiment relating to the figs 2 and 3, i.e. the header sleeve is provided with recesses 25 in the header for forming of locking tabs 27 in the ring electrode and the outer wire coil 9 is attached to the header sleeve by clamping the ring electrode around it and a clamping sleeve 26.

The header 14 according to the invention will accordingly serve several purposes. On the one hand it will accommodate the helix inside the header bore 2 such that the helix may be completely contained inside the bore during inserting of the lead into the body and may be screwed out from the distal end and into the tissue for fixating the lead to an organ. Moreover, the header will provide for bearing of the shaft 4 and allow rotation as well as longitudinal displacement of the shaft. The header also provides, as already described, for means for quick fixating of the ring electrode to the header. Finally, the header provides for electrical insulation of the inner wire coil 6, shaft 4 and helix 3 from the ring electrode 16 and the outer wire coil 9. Since the ring electrode 16 is supported by the header 14 and the inner sleeve 12, which is extended over the outside of a proximal portion of the header, no additional insulating components are required between these parts.

Accordingly, a medical implantable lead according to the invention, will facilitate assembling of the lead and reduces the number of components required. For example, no separate coupling member is required and since the header is manufactured of an electrically insulating material, no additional insulating layer, as the layer 13 in fig 1, is required on the outside of the header. Moreover, the lead can be manufactured with an increased precision which reduces production yield losses. All these factors allows for manufacturing of medical implantable leads to a lower cost.

## Claims

1. A medical implantable lead for monitoring and/or controlling of an organ inside a human or animal body, comprising two electrical conductors (6, 9), each connected to a respective electrode for receiving or transmitting of electrical signals from or to the organ, and a tubular header (14) in a distal end of the lead, wherein one electrode (3) is positioned in the outermost distal end of the lead whereas the other is a ring formed electrode (16), which is mounted on the outside of the header by means of a coupling, whereby the coupling and the header (14) are integrated into one unitary piece of an electrically insulating material such that the header is formed with a coupling portion adapted for a quick fixating connection of the ring electrode (16) to the header, **characterized in that** said coupling portion is constituted by recesses (25) in the outer surface of the header, that the quick fixating connection is a clamp connection, and that the clamp connection is carried out by punching locking tabs (27) from the ring electrode (16), which engage in said recesses (25).

2. A medical implantable lead according to claim 1, **characteri-** - **zed** in that the ring electrode (16) is formed with a sleeve portion (20), which reaches over an electrical conductor (9) and is connected to the conductor by means of a clamp connection.

3. A medical implantable lead according to claim 2, **characteri-** - **zed** in that the connection of the ring electrode (16) to the conductor (9) and the connection of the conductor to the header (14) is performed with the same clamp connection.

4. A medical implantable lead according to any of the preceding claims, **characterized in that** besides the quick fixating connection, an adhesive is used for increased bonding strength and fluid sealing.

5. A method for manufacturing of a medical implantable lead of the type adapted for monitoring and/or controlling of an organ inside a human or animal body and comprising two electrical conductors (6, 9), each connected to a respective electrode for receiving or transmitting of electrical signals from or to the organ, and a tubular header (14) in a distal end of the lead, wherein one electrode (3) is positioned in the outermost distal end of the lead whereas the other is a ring formed electrode (16), which is mounted on the outside of the header by means of a coupling, comprising the steps of:
integrating the coupling and the header (14) into one unitary piece of an electrically insulating material such that the header is formed with a coupling portion;
forming the coupling portion such that it is adapted for a quick fixating connection of the ring electrode to the header, whereby said forming of the coupling portion includes forming recesses (25) in the outer surface of the header such that said formed coupling portion is constituted by said recesses, and
forming the quick fixating connection as a clamp connection, whereby the clamp connection is formed by punching locking tabs (27) in the ring electrode (16), which engage in said recesses (25) in the outer surface of the header (14).

6. A method according to claim 5, comprising the further step of connecting the ring electrode (16) to a conductor (9) in the lead by means of a clamp connection.

7. A method according to claim 6, comprising the further step of connecting the ring electrode (16) to the conductor (9) in the lead in the same operation as connecting the ring electrode to the header (14).

8. A method according to claim 6 or 7, comprising the further step of connecting the conductor (9) in the lead to the header (14) by means of the same clamp connection as connecting the ring electrode (16) to the conductor.

9. A method according to any of the claims 5 to 8, comprising the further step of using an adhesive between the header (14) and the ring electrode (16) for achieving an increased bonding strength and fluid tight seal.

## Patentansprüche

1. Medizinische implantierbare Leitung zum Überwachen und/oder Steuern eines Organs innerhalb des Körpers eines Menschen oder Tieres, welche zwei elektrische Leiter (6, 9), die jeweils mit einer entsprechenden Elektrode zum Empfangen oder Senden elektrischer Signale von oder zu dem Organ verbunden sind, und ein röhrenförmiges Kopfteil (14) an einem distalen Ende der Leitung umfasst, wobei eine Elektrode (3) am äußersten distalen Ende der Leitung angeordnet ist, während es sich bei der anderen um eine ringförmige Elektrode (16) handelt, die mittels eines Kupplungsstücks an der Außenseite des Kopfteils angebracht ist, wobei das Kupplungsstück und das Kopfteil (14) zu einem einheitlichen Stück eines elektrisch isolierenden Materials integriert sind, derart, dass das Kopfteil mit einem einheitlichen Kupplungsabschnitt ausgebildet ist, welcher für eine schnelle fixierende Verbindung der Ringelektrode (16) an dem Kopfteil geeignet ist, **dadurch gekennzeichnet, dass** der Kupplungsabschnitt durch Aussparungen (25) in der Außenfläche des Kopfteils aufgebaut ist, dass es sich bei der schnellen fixierenden Verbindung um eine Klemmverbindung handelt und dass die Klemmverbindung durch durchstoßende Verriegelungslaschen (27) von der Ringelektrode (16) erfolgt, welche in die Aussparungen (25) eingreifen.

2. Medizinische implantierbare Leitung nach Anspruch 1, **dadurch gekennzeichnet, dass** die Ringelektrode (16) mit einem Mantelabschnitt (20) ausgebildet ist, welcher über einen elektrischen Leiter (9) reicht und über eine Klemmverbindung mit dem Leiter verbunden ist.

3. Medizinische implantierbare Leitung nach Anspruch 2, **dadurch gekennzeichnet, dass** die Verbindung der Ringelektrode (16) mit dem Leiter (9) und die Verbindung des Leiters mit dem Kopfteil (14) mit derselben Klemmverbindung erfolgt.

4. Medizinische implantierbare Leitung nach einem der vorhergehenden Ansprüche, **dadurch gekennzeichnet, dass** außer der schnellen fixierenden Verbindung ein Klebstoff verwendet wird, um eine verbesserte Bindungsstärke und eine Abdichtung gegen Fluide zu erreichen.

5. Verfahren zur Herstellung einer medizinischen implantierbaren Leitung der Art, die zum Überwachen und/oder Steuern eines Organs innerhalb des Körpers eines Menschen oder Tieres geeignet ist, und welche zwei elektrische Leiter (6, 9), die jeweils mit einer entsprechenden Elektrode zum Empfangen oder Senden elektrischer Signale von oder zu dem Organ verbunden sind, und ein röhrenförmiges Kopfteil (14) an einem distalen Ende der Leitung umfasst, wobei eine Elektrode (3) am äußersten distalen Ende der Leitung angeordnet ist, während es sich bei der anderen um eine ringförmige Elektrode (16) handelt, die mittels eines Kupplungsstücks an der Außenseite des Kopfteils angebracht ist, wobei das Verfahren die folgenden Schritte umfasst:
Integrieren des Kupplungsstücks und des Kopfteils (14) zu einem einheitlichen Stück eines elektrisch isolierenden Materials, derart, dass das Kopfteil mit einem Kupplungsabschnitt ausgebildet ist;
Bilden des Kupplungsabschnitt derart, dass er für eine schnelle fixierende Verbindung der Ringelektrode an dem Kopfteil geeignet ist, wobei das Bilden des Kupplungsabschnitts das Bilden von Aussparungen (25) in der Außenfläche des Kopfteils umfasst, derart, dass der gebildete Kupplungsabschnitt durch die Aussparungen aufgebaut ist; und
Bilden der schnellen fixierenden Verbindung als Klemmverbindung, wobei die Klemmverbindung durch durchschlagende Verriegelungslaschen (27) in der Ringelektrode (16) gebildet wird, welche in die Ausnehmungen (25) in der Außenfläche des Kopfteils (14) eingreifen.

6. Verfahren nach Anspruch 5, welches den weiteren Schritt des Verbindens der Ringelektrode (16) mit einem Leiter (9) in der Leitung mittels einer Klemmverbindung umfasst.

7. Verfahren nach Anspruch 6, welches den weiteren Schritt des Verbindens der Ringelektrode (16) mit dem Leiter (9) in der Leitung in demselben Vorgang wie dem des Verbindens der Ringelektrode mit dem Kopfteil (14) umfasst.

8. Verfahren nach Anspruch 6 oder 7, welches den weiteren Schritt des Verbindens des Leiters (9) in der Leitung mit dem Kopfteil (14) mittels derselben Klemmverbindung wie beim Verbinden der Ringelektrode (16) mit dem Leiter umfasst.

9. Verfahren nach einem der Ansprüche 5 bis 8, welches den weiteren Schritt des Verwendens eines Klebstoffs zwischen dem Kopfteil (14) und der Ringelektrode (16) umfasst, um eine verbesserte Bindungsstärke und eine Abdichtung gegen Fluide zu erreichen.

## Revendications

1. Fil médical implantable destiné à contrôler et/ou commander un organe à l'intérieur d'un corps humain ou animal, comprenant deux conducteurs électriques (6, 9), reliés chacun à une électrode respective pour recevoir ou transmettre des signaux électriques venant de ou envoyés à l'organe, et un embout tubulaire (14) dans une extrémité distale du fil, dans lequel une électrode (3) est positionnée dans l'extrémité distale la plus à l'extérieur du fil tandis que l'autre est une électrode de forme annulaire (16) qui est montée à l'extérieur de l'embout au moyen d'un raccord, le raccord et l'embout (14) étant intégrés d'un seul tenant dans une pièce en matériau électriquement isolant de telle façon que l'embout est formé avec une partie de raccord adaptée pour une connexion à fixation rapide de l'électrode annulaire (16) à l'embout, **caractérisé en ce que** ladite partie de raccord est constituée par des creux (25) dans la surface extérieure de l'embout, **en ce que** la connexion à fixation rapide est une connexion à serrage et **en ce que** la connexion à serrage est réalisée en défonçant des languettes de verrouillage (27) de l'électrode annulaire (16) qui s'engagent dans lesdits creux (25).

2. Fil médical implantable selon la revendication 1, **caractérisé en ce que** l'électrode annulaire (16) est formée avec une partie de manchon (20) qui passe par dessus un conducteur électrique (9) et qui est reliée au conducteur au moyen d'une connexion à serrage.

3. Fil médical implantable selon la revendication 2, **caractérisé en ce que** la connexion de l'électrode annulaire (16) au conducteur (9) et la connexion du conducteur à l'embout (14) sont réalisées au moyen de la même connexion à serrage.

4. Fil médical implantable selon l'une quelconque des revendications précédentes, **caractérisé en ce que**, en plus de la connexion à fixation rapide, un adhésif est utilisé pour augmenter la résistance de l'assemblage et l'étanchéité aux fluides.

5. Procédé d'un fabrication d'un fil médical implantable du type adapté pour contrôler et/ou commander un organe à l'intérieur d'un corps humain ou animal et comprenant deux conducteurs électriques (6, 9), reliés chacun à une électrode respective pour recevoir ou transmettre des signaux électriques venant de ou envoyés à l'organe, et un embout tubulaire (14) dans une extrémité distale du fil, dans lequel une électrode (3) est positionnée dans l'extrémité distale la plus à l'extérieur du fil tandis que l'autre est une électrode de forme annulaire (16) qui est montée à l'extérieur de l'embout au moyen d'un raccord, ledit procédé comprenant les étapes consistant à :
intégrer le raccord et l'embout (14) d'un seul tenant dans une pièce en matériau électriquement isolant de telle façon que l'embout est formé avec une partie de raccord ;
former la partie de raccord de façon qu'elle soit adaptée à une connexion à fixation rapide de l'électrode annulaire à l'embout, ladite formation de la partie de raccord comprenant la formation de creux (25) dans la surface extérieure de l'embout de telle façon que lesdits creux constituent ladite partie de raccord formée ; et
réaliser la connexion à fixation rapide sous la forme d'une connexion à serrage, ladite connexion à serrage étant réalisée en défonçant des languettes de verrouillage (27) de l'électrode annulaire (16) qui s'engagent dans lesdits creux (25) de la surface extérieure de l'embout (14).

6. Procédé selon la revendication 5, comprenant l'étape supplémentaire consistant à relier l'électrode annulaire (16) à un conducteur (9) du fil au moyen d'une connexion à serrage.

7. Procédé selon la revendication 6, comprenant l'étape supplémentaire consistant à relier l'électrode annulaire (16) au conducteur (9) du fil dans la même opération que celle consistant à relier l'électrode annulaire à l'embout (14).

8. Procédé selon la revendication 6 ou la revendication 7, comprenant l'étape supplémentaire consistant à relier le conducteur (9) du fil à l'embout (14) au moyen de la même connexion à serrage que celle utilisée pour relier l'électrode annulaire (16) au conducteur.

9. Procédé selon l'une quelconque des revendications 5 à 8, comprenant l'étape supplémentaire consistant à utiliser un adhésif entre l'embout (14) et l'électrode annulaire (16) pour obtenir une plus grande résistance de l'assemblage et une étanchéité aux fluides.
